(19) **Europäisches Patentamt**
**European Patent Office**
**Office européen des brevets**

(11) **EP 2 811 846 B2**

(12) **NEW EUROPEAN PATENT SPECIFICATION**
After opposition procedure

(45) Date of publication and mention
of the opposition decision:
**19.01.2022 Bulletin 2022/03**

(45) Mention of the grant of the patent:
**30.11.2016 Bulletin 2016/48**

(21) Application number: **13702184.6**

(22) Date of filing: **22.01.2013**

(51) International Patent Classification (IPC):
*A23L 33/17* (2016.01)          *A23L 2/54* (2006.01)
*A23L 2/68* (2006.01)          *A61K 9/50* (2006.01)
*A23P 10/30* (2016.01)          *A23L 29/231* (2016.01)
*A23L 33/12* (2016.01)

(52) Cooperative Patent Classification (CPC):
(C-Sets available)
**A61K 9/5052; A23L 2/54; A23L 2/68; A23L 29/231;**
**A23L 33/12; A23L 33/17; A23P 10/30;**
**A61K 9/5036;** A23V 2002/00          (Cont.)

(86) International application number:
**PCT/US2013/022550**

(87) International publication number:
**WO 2013/119384 (15.08.2013 Gazette 2013/33)**

(54) **ACIDIC AQUEOUS PRODUCT COMPRISING OIL-CONTAINING MICROCAPSULES AND METHOD FOR THE MANUFACTURE THEREOF**

SAURES WÄSSRIGES PRODUKT MIT ÖLHALTIGEN MIKROKAPSELN UND VERFAHREN ZU SEINER HERSTELLUNG

PRODUIT AQUEUX ACIDE COMPRENANT DES MICROCAPSULES CONTENANT DE L'HUILE ET SON PROCÉDÉ DE FABRICATION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **08.02.2012 US 201213368542**

(43) Date of publication of application:
**17.12.2014 Bulletin 2014/51**

(73) Proprietor: **Pepsico, Inc.**
**Purchase, NY 10577 (US)**

(72) Inventors:
• **GIVEN, JR., Peter, S.**
**Ridgefield, CT 06877 (US)**
• **TROMP, Robert, Hans**
**3524 Utrecht CN (NL)**

(74) Representative: **Maiwald Patent- und**
**Rechtsanwaltsgesellschaft mbH**
**Elisenhof**
**Elisenstraße 3**
**80335 München (DE)**

(56) References cited:
EP-A1- 0 797 925          EP-A1- 2 292 102
WO-A1-2007/034213          WO-A1-2009/070010

WO-A1-2009/080702          WO-A2-2007/120500
WO-A2-2008/085997          CN-A- 1 098 874
CN-A- 1 345 588          US-A1- 2009 004 333
US-A1- 2009 004 333          US-A1- 2009 061 048

• Stephen Daniells: "Pectin-protein combo may extend ingredient microencapsulation", Food navigator.com , 24 June 2010 (2010-06-24), XP002697207, Retrieved from the Internet: URL:http://www.foodnavigator.com/Science-N utrition/Pectin-protein-combo-may-extend-i ngredient-microencapsulation [retrieved on 2013-05-16]
• ONWULATA et al.: Whey processing, functionality and health benefits, 2008, pages 63,64,76,77,78-80,90-93-114,115, ISBN: 978-0-8138-0903-8
• KRIS-ETHERTON et al.: "Fish Consumption, Fish Oil, Omega-3 Fatty Acids, and Cardiovascular Disease; Cir- culation", Circulation, vol. 106, 2002, pages 2747-2757,
• G. Houen: "The Solubility of proteins in Organic Solvents", Acta Chemica Scandinavia, vol. 50, 1996, pages 68-70,
• C. SANCHEZ: "Thermal aggregation of Whey Protein Isolate Containing Microparticulated or Hydrolyzed Whey Proteins", J. Agric. Food Chem., vol. 45, 1997, pages 2384-2392,

Printed by Jouve, 75001 PARIS (FR)

**(Cont. next page)**

- PILAR PUYOL: "Thermal gelation of commercial whey protein concentrate: influence of pH 4.6 insoluble protein on thermal aggregation", International Journal of Dairy Technology, vol. 52, 3 August 1999 (1999-08-03), pages 81-91,
- Walstra Pieter, Et Al.: "Protein Preparations" In: "Handbook of Dairy Science and Technology", 2006 pages 537-538,
- MONTENEGRO et al.: "Products and Applications of Biopol- ymers", Gum Arabic: More Than an Edible Emulsifier, March 2012 (2012-03), ISBN: 978-953-51-0226-7
- EUSTON et al.: "Kinetics of droplet aggregation in heated whey protein-stabilized emulsions: effect of polysaccharides", Food Hydrocolloids, vol. 16, no. 5, 2002, pages 499-505,
- SANTIPANICHWONG et al.: "Core shell biopolymer nanoparticles produced by electrostatic deposition of beet pectin onto heat denatured beta-lactoglobulin aggregates", Journal of Food Science, vol. 73, no. 6, 2008, pages 23-30,
- YE et al.: "Complexation between milk proteins and polysaccharides via electrostatic interaction: principles and applications - a review", International Journal of Food Science and Technology, vol. 43, 2008, pages 406-415,
- GUZEY et al.: "Formation, stability and properties of multilayer emulsions for application in the food industry", Advances in Colloid and Interface Science, vol. 128-130, 2006, pages 227-248,
- ONWULATA et al.: Whey processing, functionality and health benefits, 2008, pages 63-64, ISBN: 978-0-8138-0903-8

(52) Cooperative Patent Classification (CPC): (Cont.)

C-Sets
A23V 2002/00, A23V 2200/224, A23V 2250/1882, A23V 2250/5072, A23V 2250/50722, A23V 2250/54, A23V 2300/08

**Description**

TECHNICAL FIELD OF THE INVENTION

The present invention relates to the method as claimed in claim 1. It is generally in the field of protecting a hydrophobic substance in an acidic aqueous system, more particularly microcapsules containing hydrophobic substances in acidic aqueous systems such as food products.

BACKGROUND OF THE INVENTION

Certain hydrophobic substances are desirable as ingredients in food products, such as in, for example, beverages. In some cases such a hydrophobic substance does not have an acceptable taste or taste profile or is not sufficiently stable in an acidic environment. Examples of such hydrophobic substances include omega-3 fatty acids, water-insoluble flavorants, water-insoluble vitamins, etc. Certain hydrophobic substances have been discovered to have beneficial health effects. For example, omega-3 and omega-6 fatty acids form an important part of the human diet. Eicosapentaenoic acid (EPA) and docosahexaenoic acid (DHA), long-chain forms of omega-3 fatty acids, are understood in many cases to support brain and cardiovascular health and functions, amongst other health benefits. It has been suggested that consumption of omega-3 fatty acids should be increased.

Previously, hydrophobic substances were incorporated directly into an aqueous system as a solution (with a compatible solvent), an extract, an emulsion, or a micellular dispersion (a so-called microemulsion). While all of these approaches serve to disperse the hydrophobic substance in an aqueous system, they do not provide extended protection against hydrolysis and oxidation. Commercially available fish oils can be high in omega-3 fatty acids, and in some cases are "encapsulated," but these commercially available fish oils have not proven adequately stable in all food contexts, e.g., physically or taste-stable in acidic food products. This can result in negative changes to the food product, such as unpleasant fishy flavors and aromas after ingestion, particularly a fishy aftertaste caused by belching fish oil from the stomach. Additionally, omega-3 fatty acids, as well as many water-insoluble flavorants, water-insoluble, vitamins, etc. are unstable to degradation, e.g., by oxidation or hydrolysis, when exposed to air, water and/or light.

It would be desirable to provide edible compositions suitable for use in food products, which compositions incorporate one or more hydrophobic substances. It also would be desirable to provide food products incorporating such edible compositions. The method as claimed provides an aqueous dispersion of microcapsules that can reduce or eliminate the unpleasant taste and odor of the one or more incorporated hydrophobic substances when used as an ingredient in a food product suitable for consumption by a human or animal and that provide hydrophobic substances in a stable form for use in food products. In at least some embodiments the hydrophobic substance is stable to oxidation and hydrolysis during the shelf life of the food product. In at least some embodiments the hydrophobic substance is stable to oxidation and hydrolysis in an acidic food product, e.g. a food product at pH less than pH 5.0 and in some cases less than pH 3.5. Additional features and advantages of some or all of the food products disclosed here will be apparent to those who are skilled in food technology given the benefit of the following summary and description of non-limiting examples.

A microencapsulated oil product and a method of making the same is disclosed in US 2009/0004333 A1.

Microcapsules containing salts for food products are disclosed in EP 2292102 A1.

SUMMARY

Aspects of the disclosure are directed to delivery systems for hydrophobic substances which may be incorporated into food products such as, for example, an acidic food product. By encapsulating a hydrophobic substance in microcapsules formed from protein aggregates and negatively charged polymers, one or more negative effects (e.g., oxidation, off flavor, unpleasant aroma, etc.) that would otherwise occur to the hydrophobic substance over time, e.g., during its incorporation into a food product, during shipment, storage, or the like, can be reduced or eliminated.

In one aspect of the disclosure, a food product comprising an aqueous dispersion of microcapsules is provided wherein the microcapsules have at least one hydrophobic substance and a layer around the at least one hydrophobic substance. The layer comprises protein aggregates and negatively charged polymers having blockwise charge distribution. The food product comprises at least a second food ingredient with the aqueous dispersion of microcapsules.

The protein aggregates used in the method as claimed comprise or consist essentially of denatured globular protein that is at least 50 wt.% denatured or that is at least 70 wt.% denatured. Certain embodiments comprise or consist essentially of denatured globular proteins that are at least 80 wt.% denatured. In certain embodiments the denatured globular proteins may be selected from denatured whey protein, denatured egg protein, denatured soy protein, denatured lupine protein, denatured rice protein, denatured pea protein, denatured wheat protein, and combinations of any of them. In certain embodiments the at least one hydrophobic substance may comprise lipids, water-insoluble vitamins, water-insoluble sterols, water-insoluble flavonoids, flavors, and essential oils. In certain embodiments the negatively charged polymer may comprise pectin, which may be selected from a high ester pectin, a low ester pectin, an amidated pectin, and combinations of any of them. In some embodiments, the at least one hydrophobic substance comprises an omega-3 fatty acid, the protein aggregates comprise whey protein, and the negatively charged polymer comprises a high methyl ester pectin. In a certain embodiment of the disclosure the food product is a beverage, and may be an acidic beverage.

A method as claimed is provided for preparing an aqueous dispersion of microcapsules comprised in a food product, wherein the food product has a pH of 1.0 to 5.5 and is a carbonated soda beverage. The method comprises a) preparing a protein solution comprising protein and water; b) heating the protein solution to form a solution of protein aggregates; c) combining the solution of protein aggregates and a hydrophobic substance, and homogenizing to form an emulsion; d) adjusting the pH of the emulsion to pH 4.0 to 5.0; e) adding a negatively charged polymer having blockwise charge distribution to the emulsion and homogenizing; and f) producing microcapsules, comprising allowing the negatively charged polymer and the protein aggregates to accumulate at the interface of the hydrophobic substance and the water.

In certain embodiments of the disclosure, the solution of the protein is heated at a temperature of 60° to 200°C for a period of time defined by the formula t=10,000/(T-59), where t is the duration in minutes and T is the temperature in degree centigrade. In certain embodiments the pH of the emulsion of protein aggregates and at least one hydrophobic substance is adjusted with an acidulant to a pH of 4.0 to 5.0. In some embodiments the emulsion of protein aggregates, at least one hydrophobic substance, and pectin comprises 0.01 wt.% hydrophobic substance, 0.01-5 wt.% protein aggregates, and 0.001-1 wt.% negatively charged polymer. In some embodiments the negatively charged polymer comprises pectin.

In an aspect of the disclosure, a microcapsule is provided including at least one hydrophobic substance and a layer around the at least one hydrophobic substance, wherein the layer comprises protein aggregates and a negatively charged polymer having blockwise charge distribution.

In an aspect of the disclosure, an aqueous dispersion of microcapsules is provided wherein the microcapsules include at least one hydrophobic substance and a layer around the at least one hydrophobic substance and wherein the layer comprises protein aggregates and a negatively charged polymer having blockwise charge distribution.

In an aspect of the disclosure, a food product comprising microcapsules is provided. The microcapsules include at least one hydrophobic substance and a layer around the at least one hydrophobic substance, wherein the layer comprises protein aggregates and pectin.

In an aspect of the disclosure, an aqueous dispersion of microcapsules comprised in a food product, wherein the food product has a pH of 1.0 to 5.5 and is a carbonated soda beverage, is produced by a method as claimed comprising: a) preparing a protein solution comprising water and protein; b) heating the protein solution to form a solution of protein aggregates; c) combining the solution of protein aggregates and a hydrophobic substance, and homogenizing to form an emulsion; d) adjusting the pH of the emulsion to pH 4.0 to 5.0; e) adding a negatively charged polymer having blockwise charge distribution to the emulsion; and f) allowing the negatively charged polymer and the protein aggregates to accumulate at the interface of the hydrophobic substance and the water, thereby producing microcapsules.

In an aspect of the disclosure, an aqueous dispersion of microcapsules is provided wherein the microcapsules comprise at least one hydrophobic substance comprising omega-3 fatty acids, an interface layer around the at least one hydrophobic substance wherein the interface layer comprises protein aggregates comprising denatured whey protein that is at least 50 wt.% denatured and pectin.

In at least certain embodiments the microcapsules disclosed here (also referred to here in the alternative and interchangeable as oil-containing microcapsules, microcapsules containing hydrophobic substance, protein aggregates and negatively charged polymers, such as pectin, based microcapsules, etc.) and food products incorporating them as an ingredient have been found to have unanticipated, desirable properties. For example, in certain such embodiments, the protein aggregates and negatively charged polymer based microcapsules can remain suspended in aqueous systems, e.g. beverages, beverage concentrates, etc., for a surprisingly long period of time. In certain such embodiments the protein aggregates and negatively charged polymer based microcapsules can remain suspended in acidic aqueous systems, e.g., beverages, beverage concentrates, etc. having a pH value less than pH 5.0 and in some cases less than pH 3.5, for a surprisingly long period of time. Furthermore, it was found that in at least some embodiments the protein aggregates and negatively charged polymer containing interface layer effectively protects the hydrophobic substance in the microcapsules against oxidation and/or hydrolysis, etc.

These and other aspects, advantages and features of the present invention here disclosed will become more apparent through reference to the following detailed description. Furthermore, it is to be understood that the features and elements of the various embodiments described here are not mutually exclusive and of such features, with or without other features and elements. This disclosure includes other embodiments having any other combination or permutation.

BRIEF DESCRIPTION OF THE DRAWINGS

In the drawings, like reference characters generally refer to the same parts throughout the different views. Also, the drawing is not necessarily to scale, emphasis instead generally being placed upon illustrating the principles of the invention. In the following description, various embodiments of the present invention are described with reference to the following drawing, in which:

Figure 1 is a schematic of a microcapsule of the present invention.

DETAILED DESCRIPTION OF THE INVENTION

Various examples and embodiments of the inventive subject matter disclosed here are possible and will be apparent to the person of ordinary skill in the art, given the benefit of this disclosure. In this disclosure reference to "certain exemplary embodiments" (and similar phrases) means that those embodiments are merely non-limiting examples of the inventive subject matter and that there likely are other alternative embodiments which are not excluded. Unless otherwise indicated or unless otherwise clear from the context in which it is described, alternative elements or features in the embodiments and examples below and in the Summary above are interchangeable with each other. That is, an element described in one example may be interchanged or substituted for one or more corresponding elements described in another example. Similarly, optional or non-essential features disclosed in connection with a particular embodiment or example should be understood to be disclosed for use in any other embodiment of the disclosed subject matter. More generally, the elements of the examples should be understood to be disclosed generally for use with other aspects and examples of the devices and methods disclosed here. A reference to a component or ingredient being operative, i.e., able to perform one or more functions, tasks and/or operations or the like, is intended to mean that it can perform the expressly recited function(s), task(s) and/or operation(s) in at least certain embodiments, and may well be operative to perform also one or more other functions, tasks and/or operations. While this disclosure includes specific examples, including presently preferred modes or embodiments, those skilled in the art will appreciate that there are numerous variations and modifications within the spirit and scope of the invention as set forth in the appended claims. Each word and phrase used in the claims is intended to include all its dictionary meanings consistent with its usage in this disclosure and/or with its technical and industry usage in any relevant technology area. Indefinite articles, such as "a," and "an" and the definite article "the" and other such words and phrases are used in the claims in the usual and traditional way in patents, to mean "at least one" or "one or more." The word "comprising" is used in the claims to have its traditional, open-ended meaning, that is, to mean that the product or process defined by the claim may optionally also have additional features, elements, etc. beyond those expressly recited. The phrase "consisting essentially of" is used to signal that the product or process defined necessarily includes the listed ingredients and is open to unlisted ingredients that do not materially affect the basic and novel properties of the invention.

Aspects of the disclosure relate to microcapsules disclosed here for hydrophobic substances, which provide a stable composition suitable for inclusion in food products, that is the microcapsules are stable for shelf-storage, for use in making food products, and for shelf-storage when included in acidic food products. The microcapsules reduce or eliminate the unpleasant taste and odor of many hydrophobic substances such as fish oil, and reduce degradation, e.g. by oxidation or hydrolysis, of unstable hydrophobic substances. The microcapsules may be incorporated into a food product associated with health benefits, for example orange juice, to provide enhanced nutritional value. Additionally, the microcapsules may be incorporated into food products, for example carbonated soft drinks. By encapsulating such hydrophobic substances in microcapsules, possible negative visual and physical changes to the food product may be reduced or avoided. The resulting food product is appealing to the consumer, as well as being stable and having an adequate shelf life.

As used here an "aqueous dispersion" is defined as particles distributed throughout a medium of liquid water, e.g., as a suspension, a colloid, an emulsion, a sol, etc. The medium of liquid water may be pure water, or may be a mixture of water with at least one water-miscible solvent, such as, for example, ethanol or other alcohols, propylene glycol, glycerin, dimethylsulfoxide, dimeth-ylformamide, etc. In certain embodiments there may be a substantial concentration of water-miscible solvent in the aqueous dispersion of the microcapsules, such as, between about 1% and about 20% by volume, for example 5%, 10%, or 15%. In other embodiments the microcapsules are diluted into a food product and the concentration of water-miscible solvent is negligible.

As used here "microcapsule" is defined as a clearly identifiable discrete particle containing one or more hydrophobic substances, e.g. oil, water-insoluble vitamins, flavors, etc. that are enveloped by an interface layer that separates said hydrophobic substances from the environment surrounding the particle. In certain embodiments there may be clearly identifiable discrete clusters (e.g. agglomerates) of the aforementioned particles.

As used here a "hydrophobic substance" refers to a water immiscible material such as an oil, a lipid, a water-insoluble vitamin (e.g. $\alpha$-tocopherol), a water-insoluble sterol, a water-insoluble flavonoid, a flavor or an essential oil. The oil employed in accordance with the present invention can be a solid, a liquid or a mixture of both.

As used here a "lipid" encompasses any substance that contains one or more fatty acid residues, including free fatty acids. Thus, the term "lipid" encompasses, for instance, triglycerides, diglycerides, monoglycerides, free fatty acids, phospholipids or a combination of any of them.

As used here a "fatty acid" encompasses free fatty acids as well as fatty acid residues. Whenever reference is made here to a weight percentage of fatty acids, this weight percentage includes free fatty acids as well as fatty acid residues (e.g. fatty acid residues contained in triglycerides). Further, as used here a "polyunsaturated fatty acid" (PUFA) encompasses any fatty acid containing 2 or more double bonds in the carbon chain.

As used here "protein" refers to a polymer built from amino acids arranged in a chain and joined together by peptide bonds between the carboxyl and amino groups of adjacent amino acid residues. Typically, the protein contains at least 10 amino acid residues. The protein employed in accordance with the present invention can be, for instance, an intact naturally occurring protein, a protein hydrolysate or a synthesised protein. Further, as used here, a "globular protein" refers to a protein having a close to spherical tertiary structure wherein the molecule's apolar (hydrophobic) amino acids are oriented towards the molecule's interior and the molecule's polar (hydrophilic) amino acids are oriented outwards, allowing dipole-dipole interactions with water. A protein's polar side groups tend to exert attractive forces on other polar groups of atoms within the protein molecule, or on polar molecules in the protein's surroundings. Similarly, non polar side groups exert attractive forces (of a different nature) on other non-polar side chains within the protein. The shape assumed by a globular protein molecule tends to maximize both types of attractive forces, whereby non polar side chains "point" inward and interact with one another and polar side chains are oriented outward such that they are exposed to adjacent polar water molecules. In some embodiments upon heat treatment these attractive forces become overcome by heat energy, and the protein begins to unfold. The unfolding of the protein is known here as "denaturation". A protein that is only partially unfolded, i.e., has lost some tertiary and secondary structure, is "partially denatured". As used here a "denatured protein" refers to a protein that is denatured to the point of becoming an activated protein. A denatured protein may be, for example, at least 50%, at least 70%, or at least 80% unfolded.

As used here "blockwise charge" refers to blocks of an ionic charge located along a polymer creating areas having a higher charge density than the polymer as a whole. For example, a negatively charged polymer having blockwise charge distribution may have blocks of a higher negative ionic charge, which may be due to clusters of acid groups distributed along the polymer, thereby causing the negative charge to be distributed along the polymer in blocks rather than uniformly.

As used here an "interface layer" is the layer that separates the one or more hydrophobic substances in the microcapsule from the surrounding environment (e.g. an aqueous liquid or a gaseous atmosphere). As used here a "proteinaceous interface layer" is an interface layer that, water excluded, contains at least 25 wt.%, preferably at least 50 wt.% of protein and/or protein derivatives, such as protein aggregates. Further, as used here "the interface layer predominantly consists of protein aggregates and pectin" means that the interface layer, water excluded, mainly consists of protein aggregates and pectin. The composition of the interface layer may suitably be determined by, if necessary, isolating the microcapsules from other product constituents, followed by the removal of water and oil (including any components entrapped in the oil) and analysis of the residue so obtained.

In certain embodiments an aqueous solution is prepared comprising at least one protein. The aqueous solution may comprise, for example, 5 to 10 wt.% protein in water or in water with other solvents, optionally with other ingredients. The at least one protein may comprise or consist essentially of, for example, whey protein, such as, beta-lactoglobulin, alpha-lactalbumin, whey protein isolate, whey protein concentrate, egg protein, lupine protein, soy protein, rice protein, pea protein, wheat protein, or a combination of any of them. In certain embodiments the at least one protein comprises globular proteins having a molecular weight of, for example, at least 5 kDa, or alternatively, at least 10 kDa. In some embodiments, for example, when the molar mass of the globular protein is larger than 20 kDa, the aqueous solution may be subjected to high-shear mixing. The aqueous solution of at least one protein is heated at a temperature within the range of 60 to 200°C for at least a period of time equal to t, which period of heating t is governed by the formula:

$$t = \frac{10,000}{(T-59)}$$

wherein: t = duration of heating (in minutes) and T = heating temperature (in °C). The temperature and time required for obtaining the minimum aggregation may vary dependent on, for example, the types of protein used, the applied shear, pH of the solution, or salts present. It is currently understood that the heat treatment of the aqueous solution of at least one protein produces an aqueous solution of protein aggregates.

The protein aggregates in the aqueous solution comprise at least one denatured globular protein. In some embodiments the at least one globular protein may only be partially denatured. In certain embodiments the at least one globular protein may be at least 50% denatured. In certain embodiments the protein aggregates comprise denatured globular proteins that are, for example, at least 50 wt.%, at least 70 wt.%, or at least 80 wt.% denatured. In certain embodiments the at least one denatured globular protein comprises denatured whey protein, denatured egg protein, denatured soy protein, denatured lupine protein, denatured rice protein, denatured pea protein, denatured wheat protein, or a combination of any of them. In some embodiments the at least one denatured globular protein is denatured whey protein. In certain embodiments the protein aggregates comprise additional components, for example, additional proteins that are capable of participating in the protein aggregates. Examples of such other proteins include egg protein, wheat protein or other proteins able to form insoluble aggregates after heating.

In certain embodiments the protein aggregates have a volume weighted average diameter within the range of, for example, 5-250 nm, 10-150 nm, or 20-100 nm. The diameter of the protein aggregates may be measured by, for example, light scattering techniques.

In certain embodiments an emulsion is prepared by combining the aqueous solution of protein aggregates with a hydrophobic substance. In certain embodiments the hydrophobic substance is, for example, an oil droplet. In certain embodiments the oil droplet is a lipophilic nutrient or a water-insoluble flavorant. In certain embodiments the protein aggregates accumulate to form an interface layer around the oil droplet.

In certain embodiments the emulsion is homogenized prior to the protein aggregates forming an interface layer. The homogenization can be performed by any suitable techniques, including, for example, a two-step homogenization process (i.e., 800 and 80 bar). After homogenization, the protein aggregates accumulate at the interface of the water and the oil forming an interface layer and thereby forming microcapsules in an aqueous dispersion. In certain embodiments an acidulant may be added after preparation of the emulsion. The acidulant may be any food grade acidulant, for example, citric acid, Glucono delta-lactone, adipate, acetic acid, phosphoric acid, tartaric acid and combinations of any of them. In some embodiments the emulsion may be treated with anti-microbials, radiation, or packaged aseptically.

In certain embodiments the lipophilic nutrients may comprise or consist essentially of, fat soluble vitamins, (e.g., vitamins A, D, E, and K), tocotrienols, carotenoids, xanthophylls, (e.g., lycopene, lutein, astaxanthin, and zeazanthin), fat-soluble nutraceuticals including phytosterols, stanols and esters thereof, Coenzyme Q10 and ubiquinol, hydrophobic amino acids and peptides, essential oils and extracts, and fatty acids. Fatty acids may include, for example, conjugated linolenic acid (CLA), omega-6 fatty acids, and omega-3 fatty acids. Suitable omega-3 fatty acids include, e.g., short-chain omega-3 fatty acids such as alpha-linolenic acid (ALA), which are derived from plant sources, for example flaxseed, and long-chain omega-3 fatty acids such as eicosapentaenoic acid (EPA) and docosahexaenoic acid (DHA). The long-chain omega-3 fatty acids can be derived from, for example, marine or fish oils. Such oils can be extracted from various types of fish or marine animals, such as anchovies, capelin, cod, herring, mackerel, menhaden, salmon, sardines, shark and tuna, or from marine vegetation, such as micro-algae, or a combination of any of them. Other sources of omega-3 fatty acids include liver and brain tissue and eggs.

In certain embodiments the water-insoluble flavorant may comprise or consist essentially of, any substance that provides a desired flavor to a food or beverage product, which does not substantially dissolve in water (e.g., non-polar, hydrophobic substances such as lipids, fats, oils, etc.). The flavorant may be a liquid, gel, colloid, or particulate solid, e.g., an oil, an extract, an oleoresin, or the like. Examples of water-insoluble flavorants include, but are not limited to, citrus oils and extracts, e.g. orange oil, lemon oil, grapefruit oil, lime oil, citral and limonene, nut oils and extracts, e.g. almond oil, hazelnut oil and peanut oil, other fruit oils and extracts, e.g. cherry oil, apple oil and strawberry oil, botanical oils and extracts, e.g., coffee oil, mint oil, vanilla oil, and combinations of any of them.

In some embodiments a negatively charged polymer having blockwise charge distribution is added to the emulsion of protein aggregates and the hydrophobic substance after homogenisation of the emulsion and after addition of an acidulant.

The negatively charged polymer consists of a negatively charged polymer having blockwise charge distribution. In some embodiments the negatively blockwise charged polymer may be, for example, pectin, carboxy methyl cellulose, modified starches (such as octenyl succinate modified starch), carrageenan, alginate, partially deacetylated xanthan, or partially deacetylated gellan, or a combination of any of them.

In certain embodiments the negatively charged polymer may comprise or consist essentially of pectin. In some embodiments the pectin may be a negatively charged pectin having blockwise charge distribution. In certain embodiments pectin may be selected, for example, from the group comprising a high ester (HM) pectin ($\geq$50% esterification), a low ester (LM) pectin ($\leq$50% esterification), an amidated pectin, and combinations of any of them. In some embodiments the pectin is a high methyl ester pectin. In certain embodiments the pectin is more than 65% esterified. In certain embodiments the pectin is a citrus pectin. In certain embodiments the pectin has a molecular weight of, for example, 60,000-200,000 g/mol, or alternatively, 100,000-150,000 g/mol.

In certain embodiments an interface layer comprising protein aggregates and pectin has a combined dry weight composition of, for example, at least 55%, or at least 70%. In certain embodiments the interface layer comprises protein aggregates and the pectin in a weight ratio of, for example, 10:1 to 1:4 or 5:1 to 1:3. It is Applicants' belief that if a microcapsule has an interface layer within this ratio, the microcapsule will have particularly high chemical and physical stability. In certain embodiments the thickness of the interface layer in at least a majority of the microcapsules is, for example, within the range of 0.005 to 10 $\mu$m, 0.05 to 5 $\mu$m, or 0.1 to 1 $\mu$m.

In certain embodiments the microcapsules are characterized by a ratio [X]:[Y] of, for example, less than 1:1.3 or less than 1:1.2, wherein [X] is the weight percentage of the protein aggregates contained in the interface layer that can be dissolved when 75 mg of the microcapsules is dispersed in 50 ml distilled water having a temperature of 5°C at any pH within the range of 3.0 to 7.0; and [Y] is the weight percentage of the protein aggregates contained in the interface layer that can be dissolved when 75 mg of the microcapsules is dispersed in 50 ml aqueous solution of 2 wt.% dithiothreitol (DTT), having a temperature of 5°C at any pH within the range of 3.0 to 7.0.

In certain embodiments the emulsion comprises, for example, 0.01-45 wt.%, or alternatively 0.01-20 wt.%, of dispersed oil; 0.001-5 wt.%, or alternatively 0.001-2 wt.% of protein aggregates; 0.001-1 wt.%, of negatively charged polymer; 45-99.99 wt.%, or alternatively 70-99.99 wt.%, of water; wherein the various constituents together represent, for example, at least 95 wt.% of the emulsion.

In certain embodiments the oil droplets contain, for example, at least 3 wt.% or, alternatively 10 wt.%, of one or more polyunsaturated fatty acids selected from omega-3 fatty acids, omega-6 fatty acids and combinations of any of them. In certain embodiments the one or more polyunsaturated fatty acids are selected from DHA, EPA, CLA, and combinations of any of them. In alternative embodiments the oil droplets contain, for example, at least 50 wt.%, at least 70 wt.%, or at least 80 wt.% of lipids.

In certain embodiments the microcapsules of the present invention have a volume weighted average diameter in the range of, for example, 0.1-100 μm, 0.3-50 μm, 0.5-30 μm, or 0.7-20 μm. In certain embodiments the oil droplets in the microcapsules have a diameter in the range of, for example, 0.01-20 μm. In other embodiments the oil droplets have a diameter in the range of 0.1-10 μm.

In certain embodiments the oil droplets represent, for example, at least 5 wt.%, at least 10 wt.%, at least 20 wt.%, or at least 35 wt.% of the microcapsule. In certain embodiments the oil droplets represent not more than 80 wt.% of the microcapsules. In certain embodiments the oil droplets typically have a melting point of, for example, less than 40°C, less than 30°C, or less than 15°C.

In certain embodiments the solution of protein aggregates is prepared at a neutral pH of, for example, about pH 6.0 to pH 7.0. In certain embodiments the emulsion formed from the protein aggregates and the at least one hydrophobic substance is adjusted to a pH of, for example, about pH 4.0 to pH 5.0. In certain embodiments after the pH of the emulsion is adjusted the pectin may be added. The aqueous dispersion containing the micro-capsules is comprised in an acidic food product that has a pH of 1.0 to pH 5.5 and is a carbonated soda beverage. In some embodiments of the disclosure the aqueous dispersion of microcapsules is added to a food product having a final pH of at least pH 3.0.

In certain embodiments the aqueous dispersion of the present invention may contain other dispersed components in addition to the microcapsules. In certain embodiments the dispersion contains less than 20 wt.% of one or more dispersed edible components, including the dispersed microcapsules.

In certain embodiments the microcapsules are not substantially additionally stabilized, for example by substantial gelling or substantial hardening of the microcapsules. In certain alternative embodiments the protein aggregates may be cross-linked, for example, by utilizing a cross-linking agent, for example, gluteraldehyde. In an alternative embodiment the protein aggregates may be cross-linked by forming disulphide bridges between the protein molecules.

In certain embodiments the aqueous dispersion of microcapsules is maintained as an aqueous dispersion. In alternative embodiments the aqueous dispersion of microcapsules is, for example, spray dried, freeze dried, drum dried, or bed dried. If maintained as an aqueous dispersion, in certain embodiments, the aqueous dispersion of microcapsules is treated to protect from microbiological growth. In certain embodiments the aqueous dispersion of microcapsules is, for example, pasteurized; aseptically packaged; treated with chemical preservatives, such as, sodium hexameta phosphate (SHMP), calcium propionate, calcium sorbate, potassium sorbate, sodium citrate, potassium citrate, calcium benzoate, sodium benzoate, potassium benzoate, sodium nitrate, sodium chloride, sulphur dioxide, natamycin, nisin, sulfites (sulfur dioxide, sodium bisulfite, potassium hydrogen sulfite, etc) or disodium EDTA; treated with acids such as citric acid, Glucono delta-lactone, adipate, phosphoric acid, acetic acid, tartaric acid, succinic acid, or HCl; carbonated; or combinations of any of them. In some embodiments the aqueous dispersion of microcapsules has minimized contact with air during production, is pasteurized after production, and is stored in a refrigerator with limited contact with light.

In certain embodiments the aqueous dispersion of microcapsules contains the dispersed microcapsules in a concentration of, for example, 0.03-10% by weight, or in some embodiments 0.1-5% by weight of the continuous aqueous phase. In certain embodiments the aqueous dispersion contains a limited amount of microcapsules and the continuous aqueous phase represents the bulk, for example, at least 70 wt.%, 80-99.9 wt.%, or 90-99.9 wt.% of the aqueous dispersion.

In certain embodiments the aqueous dispersion of microcapsules is a pourable concentrate that can be used to deliver the microcapsules to e.g. food products. In accordance with this embodiment the aqueous dispersion contains 10-50 wt.% of the microcapsules and 50-10 wt.% of the continuous aqueous phase.

In certain embodiments of the disclosure a concentrated microcapsule composition is prepared containing, for example, at least 1 wt.%, at least 5 wt.%, or at least 10 wt.% of dispersed microcapsules. In certain embodiments the concentrated microcapsule composition is combined with water and, in certain embodiments, other constituents to produce a food product. In certain embodiments the combining of the concentrated microcapsule composition with water and other constituents produces a dilution factor (final volume/volume of the concentrated microcapsule composition), for example, of at least 3, or alternatively, of at least 5. In certain embodiments the dilution factor does not exceed 1000.

In certain embodiments a desired amount of hydrophobic substance in the form of the above-described microcapsules is included in a food product. The amount of microcapsules, and hence the amount of hydrophobic substance included in the food product may vary depending on the application and desired taste characteristics of the food product. The microcapsules may be added to the food product in any number of ways, as would be appreciated by those of ordinary skill in the art given the benefit of this disclosure. In certain embodiments the microcapsules are sufficiently mixed in the food product to provide a substantially uniform distribution, for example a stable dispersion. Mixing should be accomplished such that the microcapsules are not destroyed. If the microcapsules are destroyed, oxidation of the hydrophobic substance may result. The mixer(s) can be selected for a specific application based, at least in part, on the type and amount of ingredients used, the viscosity of the ingredients used, the amount of product to be produced, the flow rate, and the sensitivity of ingredients, such as the microcapsules, to shear forces or shear stress.

Encapsulation of hydrophobic substances using the above-described microcapsules stabilizes the hydrophobic substance by protecting it from degradation by, for example, oxidation and hydrolysis. When included in an acidic food product, the microcapsules can provide a stable dispersion of hydrophobic substances over the shelf life of the food product. Factors that may effect the shelf-life of the microcapsules include the level of processing the product undergoes, the type of packaging, and the materials used for packaging the product. Additional factors that may affect the shelf life of the product include, for example, the nature of the base formula (e.g., an acidic beverage sweetened with sugar has a longer shelf-life than an acidic beverage sweetened with aspartame) and environmental conditions (e.g., exposure to high temperatures and sunlight is deleterious to ready-to-drink beverages).

The food product is a beverage product, namely a carbonated soda beverage having a pH of 1.0 to 5.5. In certain embodiments the beverage products, namely a carbonated soda beverage having a pH of 1.0 to 5.5, include ready-to-drink beverages, beverage concentrates, syrups, shelf-stable beverages, refrigerated beverages, frozen beverages, and the like. In some embodiments the beverage product, namely a carbonated soda beverage having a pH of 1.0 to 5.5, has a pH within the range below about pH 5.0, in certain embodiments a pH value within the range of about pH 1.0 to about pH 4.5, or in certain embodiments a pH value within the range of about pH 1.5 to about pH 3.8. In some embodiments the beverage product, namely a carbonated soda beverage having a pH of 1.0 to 5.5, has a pH of 3.0. Beverage products of the disclosure include, but are not limited to, e.g., carbonated and non-carbonated soft drinks, fountain beverages, liquid concentrates, fruit juice and fruit juice-flavored drinks, sports drinks, energy drinks, fortified/enhanced water drinks, soy drinks, vegetable drinks, grain-based drinks (e.g. malt beverages), fermented drinks (e.g., yogurt and kefir), coffee beverages, tea beverages, dairy beverages, and mixtures thereof. Exemplary fruit juice sources include citrus fruit, e.g. orange, grapefruit, lemon and lime, berry, e.g. cranberry, raspberry, blueberry and strawberry, apple, grape, pineapple, prune, pear, peach, cherry, mango, and pomegranate. Beverage products of the disclosure include bottle, can, and carton products and fountain syrup applications.

Certain embodiments of the disclosure of other food products include fermented food products, yogurt, sour cream, cheese, salsa, ranch dip, fruit sauces, fruit jellies, fruit jams, fruit preserves, and the like. In certain embodiments of the disclosure the food product is acidic, e.g. having a pH value within the range below about pH 5.0, in certain embodiments a pH value within the range of about pH 1.0 to about pH 4.5, or in certain embodiments a pH value within the range of about pH 1.5 to about pH 3.8. In some embodiments the food product has a pH of 3.0.

The food product may optionally include other additional ingredients. In certain embodiments additional ingredients may include, for example, vitamins, minerals, sweeteners, water-soluble flavorants, colorings, thickeners, emulsifiers, acidulants, electrolytes, antifoaming agents, proteins, carbohydrates, preservatives, water-miscible flavorants, edible particulates, and mixtures thereof. In certain embodiments other ingredients are also contemplated. In some embodiments the ingredients can be added at various points during processing, including before or after pasteurization, and before or after addition of the microcapsules.

In at least certain embodiments food products disclosed here may be pasteurized. The pasteurization process may include, for example, ultra high temperature (UHT) treatment and/or high temperature-short time (HTST) treatment. The UHT treatment includes subjecting the food or beverage product to high temperatures, such as by direct steam injection or steam infusion, or by indirect heating in a heat exchanger. Generally, after the product is pasteurized, the product can be cooled as required by the particular product composition/configuration and/or the package filling application. For example, in one embodiment, the food or beverage product is subjected to heating to about 185°F (85°C) to about 250°F (121°C) for a short period of time, for example, about 1 to 60 seconds, then cooled quickly to about 36°F (2.2°C) +/10°F (5°C) for refrigerated products, to ambient temperature for shelf stable or refrigerated products, and to about 185°F (85°C) +/- 10°F (5°C) for hot-fill applications for shelf-stable products. The pasteurization process is typically conducted in a closed system, so as not to expose the food product to atmosphere or other possible sources of contamination. In alternative embodiments other pasteurization or sterilization techniques may also be useful, such as, for example, aseptic or retort processing. In addition, multiple pasteurization processes may be carried out in series or parallel, as necessitated by the food product or ingredients.

Food products may, in addition, be post processed. In some embodiments post processing is typically carried out following addition of the microcapsules. Post processing can include, for example, cooling the product solution and filling it into container for packaging and shipping. In certain embodiments post processing may also include deaeration of the food product to less than 4.0 ppm oxygen, preferably less than 2.0 ppm and more preferably less than 1.0 ppm oxygen. In alternative embodiments deaeration and other post processing tasks may be carried out prior to processing, prior to pasteurization, prior to mixing with the microcapsules and/or at the same time as adding the microcapsules. In addition, in certain embodiments an inert gas (e.g., nitrogen or argon) headspace may be maintained during the intermediary processing of the product and final packaging. Additionally/alternatively, an oxygen or UV radiation barriers and/or oxygen scavengers could be used in the final packaging.

The following examples are specific embodiments of the present invention, but are not intended to limit it.

EXAMPLES

Example 1

*Preparation of a beverage containing an aqueous dispersion of microcapsules containing fish oil*

An aqueous dispersion of microcapsules in accordance with one exemplary embodiment of the disclosure was prepared using the following method.

90 g of whey protein isolate (WPI) was dissolved in 1000 g water. The aqueous solution was then subjected stirring using a magnetic stirrer for 2 hours. The aqueous solution was split into two separate solutions (A & B).

Solution A was kept at 68.6°C for 120 min and then cooled to room temperature. After heating, the solution contained aggregates of partly unfolded whey protein molecules. The diameter of the aggregates was between 10 and 80 nm, determined by dynamic light scattering. Further, the solution was transparent and non-turbid.

Solution B was maintained at room temperature and was not heated.

An emulsion was prepared comprising 220 g of Solution A, 580 g water and 200 g fish oil by high pressure homogenisation (800/80 bar). The emulsion contains 2.5% protein (on the basis of water and protein, i.e., prior to adding the oil) and 20% fish oil. The pH of the emulsion was adjusted with a 50% aqueous solution of citric acid to a pH of 3.0.

A second emulsion was prepared comprising 220 g of Solution B, 580 g water and 200 g fish oil by high pressure homogenisation (800/80 bar). The emulsion contains 2.5% protein and 20% fish oil. The pH of the emulsion was adjusted with a 50% aqueous solution of citric acid to a pH of 3.0.

600 grams of each emulsion was mixed with a 330 g solution of 3% high-methylated pectin (HM-pectin) and 70 g water, and homogenized at 120/20 bar, to obtain two new emulsions containing 12% fish oil, 1.5% protein and 1% pectin.

Each emulsion was added to a beverage having a pH of pH 3. The oil content of the beverages after adding the emulsion was 0.072%. Each beverage was then tested for creaming, flocculation, sedimentation, and development of taste aspects related to oxidation of fish oil over a period of 7 days at 32°C (90°F).

The beverage containing fish oil stabilized by the combination of WPI aggregates and HM-pectin showed increased stability, whereas the beverage containing fish oil, native WPI and HM-pectin showed flocculation and creaming before the end of the 7 days at 32°C (90°F), as well as the development of a taste and a flavor related to oxidation.

Example 2

*Preparation of a beverage containing an aqueous dispersion of microcapsules containing fish oil*

An aqueous solution having an aqueous dispersion of microcapsules in accordance with another exemplary embodiment of the disclosure was prepared using the following method.

60 g of ovalbumine (Sigma grade III) was dissolved in 1000 g water. The aqueous solution was then subjected to stirring using a magnetic stirrer for 2 hours. The aqueous solution was split into two separate solutions (A & B).

Solution A was kept at 80°C for 30 min and then cooled to room temperature. After heating, the solution contained aggregates of partly unfolded whey protein molecules. The diameter of the aggregates was between 10 and 80 nm, determined by dynamic light scattering. Further, the solution was transparent and non-turbid.

Solution B was maintained at room temperature and was not heated.

An emulsion was prepared comprising 330 g of Solution A, 470 g water and 200 g fish oil by high pressure homogenisation (800/80 bar). The emulsion contains 2.5% protein (on the basis of water and protein, i.e., prior to adding the oil) and 20% fish oil. The pH of the emulsion was adjusted with a 50% aqueous solution of citric acid to a pH of 3.0.

A second emulsion was prepared comprising 330 g of Solution B, 470 g water and 200 g fish oil by high pressure homogenisation (800/80 bar). The emulsion contains 2.5% protein and 20% fish oil. The pH of the emulsion was adjusted with a 50% aqueous solution of citric acid to a pH of 3.0.

600 g of each emulsion was mixed with a 330 g solution of 3% high-methylated pectin (HM-pectin) and 70 g water, and homogenized at 120/20 bar, to obtain two new emulsions containing 12% fish oil, 1.5% protein and 1% pectin.

Each emulsion was added to a beverage having a pH of pH 3. The oil content of the beverages after adding the emulsion was 0.072%. Each beverage was then tested for creaming, flocculation, sedimentation, and development of taste aspects related to oxidation of fish oil over a period of 7 days at 32°C (90°F).

The beverage containing fish oil stabilized by the combination of ovalbumin aggregates and HM-pectin showed increased stability, whereas the beverage containing fish oil, native ovalbumin and HM-pectin showed flocculation and creaming before the end of the 7 days at 32°C (90°F), as well as the development of a taste and a flavor related to oxidation.

Example 3

*Preparation of a beverage containing fish oil*

An aqueous solution having an aqueous dispersion of microcapsules in accordance with another exemplary embodiment of the disclosure was prepared using the following method.

An aqueous dispersion of microcapsules in accordance with one exemplary embodiment of the disclosures was prepared using the following method.

90 g of whey protein isolate (WPI) was dissolved in 1000 g water. The aqueous solution was then subjected to stirring using a magnetic stirrer for 2 hours. The aqueous solution was split into two separate solutions (A & B).

Solution A was kept at 68.6°C for 120 min and then cooled to room temperature. After heating, the solution contained aggregates of partly unfolded whey protein molecules. The diameter of the aggregates was between 10 and 80 nm, determined by dynamic light scattering. Further, the solution was transparent and non-turbid.

Solution B was maintained at room temperature and was not heated.

An emulsion was prepared comprising 220 g of Solution A, 580 g water and 200 g fish oil by high pressure homogenisation (800/80 bar). The emulsion contains 2.5% protein (on the basis of water and protein, i.e., prior to adding the oil) and 20% fish oil. The pH of the emulsion was adjusted with a 50% aqueous solution of citric acid to a pH of 3.0.

A second emulsion was prepared comprising 220 g of Solution B, 580 g water and 200 g fish oil by high pressure homogenisation (800/80 bar). The emulsion contains 2.5% protein and 20% fish oil. The pH of the emulsion was adjusted with a 50% aqueous solution of citric acid to a pH of 3.0.

600 grams of each emulsion was mixed with a 330 g solution of 3% low-methylated pectin (LM-pectin) and 70 g water, and homogenized at 120/20 bar, to obtain two new emulsions containing 12% fish oil, 1.5% protein and 1% pectin.

Each emulsion was added to a beverage having a pH of pH 3. The oil content of the beverages after adding the emulsion was 0.072%. Each beverage was then tested for creaming, flocculation, sedimentation, and development of taste aspects related to oxidation of fish oil over a period of 7 days at 32°C (90°F).

The beverage containing fish oil stabilized by the combination of WPI aggregates and LM-pectin showed increased stability, whereas the beverage containing fish oil, native WPI and LM-pectin showed flocculation and creaming before the end of the 7 days at 32°C (90°F), as well as the development of a taste and a flavor related to oxidation.

Example 4

*Pomposition of the microcapsules*

The beverages described in Example 1 were centrifuged at 5000 g for 60 min. A 2 ml sample was taken from the oil containing turbid layer on top of the solution by means of a pipette and freeze dried using a standard freeze drying set up. Next, the sample was analysed with respect to protein (WPI), pectin, and oil content by Infra Red spectroscopy and Size Exclusion Chromatography.

The results from both beverages, one with WPI aggregates and one with native WPI are presented in Table 1.

Table 1. Contents of freeze dried encapsulate particles in mass based percentage.

| State of protein | % protein | %pectin | % fish oil | % water |
|---|---|---|---|---|
| Native | 6.2 | 15.3 | 74.0 | 4.4 |
| Aggregates | 2.4 | 8.7 | 86.7 | 2.2 |

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims.

## Claims

1. A method for forming an aqueous dispersion of microcapsules comprised in a food product, wherein the food product has a pH of 1.0 to 5.5 and is a carbonated soda beverage, the method comprising:

   a) preparing a protein solution comprising protein and water;
   b) heating the protein solution to form a solution of protein aggregates, wherein the protein aggregates comprise denatured globular protein that is at least 50 wt.% denatured,
   c) combining the solution of protein aggregates and a hydrophobic substance, and homogenizing to form an emulsion;
   d) adjusting the pH of the emulsion of pH 4.0 to 5.0;
   e) adding a negatively charged polymer having blockwise charge distribution to the emulsion and homogenizing,
   f) producing microcapsules, comprising allowing the negatively charged polymer and the protein aggregates to accumulate at the interface of the hydrophobic substance and the water,
   wherein the microcapsules comprise:
   at least one hydrophobic substance; and

      a. an interface layer around the at least one hydrophobic substance wherein the interface layer comprises:

         i. protein aggregates obtained by heat treating an aqueous protein solution; and
         ii. a negatively charged polymer having blockwise charge distribution,

   wherein the protein aggregates comprise denatured globular protein that is at least 50 wt.% denatured.

2. The method for forming an aqueous dispersion of microcapsules of claim 1, with one or more of the following characteristics:

   - wherein the protein solution comprises globular proteins having a molecular weight of at least 5 kDa,
   - wherein the protein solution comprises 5 - 10 wt.% protein,
   - wherein heating the protein solution occurs at a temperature of 60°C to 200°C,
   - wherein homogenizing to form an emulsion is a two-step homogenization process at 800 and 80 bar.

3. The method for forming an aqueous dispersion of microcapsules according to claim 1, wherein adjusting the pH of the emulsion to a pH of 4.0 to 5.0 is performed by adding an acidulant, preferably wherein the acidulant comprises citric acid, Glucono delta-lactone, adipate, acetic acid and combinations of any of them.

4. The method for forming an aqueous dispersion of microcapsules according to claims 1, wherein the microcapsules comprise 0.01 - 45 wt.% hydrophobic substance, 0.01 - 5 wt.% protein aggregates, and 0.001 - 1 wt.% negatively charged polymer.

5. The method according to claim 1, wherein the microcapsules as defined in claim 1 comprise:

   a. at least one hydrophobic substance comprising omega-3 fatty acids;
   b. an interface layer around the at least one hydrophobic substance wherein the interface layer comprises:

      i. protein aggregates comprising denatured whey protein that is at least 50 wt.% denatured, and
      ii. pectin.

6. The method according to claim 1, wherein the aqueous dispersion as defined in claim 1 has the following characteristics:

   - wherein the protein aggregates comprise denatured globular protein selected from denatured whey proteins, denatured egg proteins, denatured soy proteins, denatured lupine proteins, denatured rice proteins, denatured pea proteins, denatured wheat proteins, and combinations of any of them.
- wherein the at least one hydrophobic substance is selected from lipids, water-insoluble vitamins, water-insoluble sterols, water-insoluble flavonoids, flavours, essential oils, and combinations of any of them,
- wherein the at least one hydrophobic substance comprises a fatty acid selected from an omega-3 fatty acid, an omega-6 fatty acid, and combinations of any of them,
- wherein the at least one hydrophobic substance comprises docosahexaenoic acid (DHA), eicosapentaenoic acid (EPA), steradonic acid, $\alpha$-Linolenic acid (ALA), conjugated linoleic acid (CLA), or a combination of any of them,
- wherein the negatively charged polymer comprises pectin, carboxy methyl cellulose, modified starches, carrageenan, alginate, partially deacetylated xanthan, partially deacetylated gellan, or a combination of any of them.

7. The method according to claim 6, wherein the aqueous dispersion as defined in claim 1 has the following characteristics:

- wherein negatively charged polymer is selected from a high ester pectin, a low ester pectin, an amidated pectin and combinations of any of them, or

wherein the negatively charged polymer comprises a high methyl ester pectin, or
wherein the negatively charged polymer comprises a citrus pectin.

8. The method of claim 6, wherein the aqueous dispersion as defined in claim 1 has the following characteristics:

- wherein the protein aggregates comprise denatured whey protein,
- wherein the protein aggregates have an average diameter of 5 - 250 nm,
- wherein the microcapsules have a volume weighted average diameter in the range of 0.1 - 100 $\mu$m,

- wherein the at least one hydrophobic substance of each of at least a majority of the microcapsules is an oil droplet having a diameter in the range of 0.01 - 20 $\mu$m,
- wherein the interface layer comprises protein aggregates and negatively charged polymer in a weight ratio with the range of 10:1 to 1:4,
- wherein the interface layer comprises protein aggregates and negatively charged polymer in an amount of at least 55% of dry matter,
- wherein the interface layer has a thickness of 0.005 - 10 $\mu$m,
- wherein the at least one hydrophobic substance comprises at least 5 wt.% of the microcapsule,
- wherein the at least one hydrophobic substance comprises omega-3 fatty acid, the protein aggregates are whey protein aggregates, and the negatively charged polymer comprises a high methyl ester pectin.

**Patentansprüche**

1. Verfahren zur Bildung einer wässrigen Dispersion von Mikrokapseln, die in einem Lebensmittelprodukt enthalten ist, wobei das Lebensmittelprodukt einen pH-Wert von 1,0 bis 5,5 aufweist und ein kohlensäurehaltiges Sodagetränk ist, wobei das Verfahren umfasst:

a) Herstellen einer Proteinlösung, die Protein und Wasser umfasst;
b) Erwärmen der Proteinlösung, um eine Lösung von Proteinaggregaten zu bilden, wobei die Proteinaggregate denaturiertes globuläres Protein, das zu mindestens 50 Gew.-% denaturiert ist, umfassen,
c) Zusammenbringen der Lösung von Proteinaggregaten und einer hydrophoben Substanz, und Homogenisieren, um eine Emulsion zu bilden;
d) Einstellen des pH-Wertes der Emulsion auf einen pH-Wert von 4,0 bis 5,0;
e) Hinzugeben eines negativ geladenen Polymers mit blockweiser Ladungsverteilung zu der Emulsion und Homogenisieren,
f) Herstellen von Mikrokapseln, umfassend das Ansammelnlassen des negativ geladenen Polymers und der Proteinaggregate an der Grenzfläche der hydrophoben Substanz und des Wassers,

wobei die Mikrokapseln umfassen:
mindestens eine hydrophobe Substanz; und

a. eine Grenzflächenschicht um die mindestens eine hydrophobe Substanz herum, wobei die Grenzflächenschicht umfasst:

i. Proteinaggregate, erhalten durch Wärmebehandlung einer wässrigen Proteinlösung; und
ii. ein negativ geladenes Polymer mit blockweiser Ladungsverteilung,

wobei die Proteinaggregate denaturiertes globuläres Protein, das zu mindestens 50 Gew.-% denaturiert ist, umfassen.

2. Verfahren zur Bildung einer wässrigen Dispersion von Mikrokapseln nach Anspruch 1, mit einem oder mehreren der folgenden Merkmale:

- wobei die Proteinlösung globuläre Proteine mit einem Molekulargewicht von mindestens 5 kDa umfasst,
- wobei die Proteinlösung 5-10 Gew.-% Protein umfasst,
- wobei das Erwärmen der Proteinlösung bei einer Temperatur von 60 °C bis 200 °C erfolgt,
- wobei das Homogenisieren, um eine Emulsion zu bilden, ein zweistufiger Homogenisierungsprozess bei 800 bar und 80 bar ist.

3. Verfahren zur Bildung einer wässrigen Dispersion von Mikrokapseln nach Anspruch 1, wobei das Einstellen des pH-Wertes der Emulsion auf einen pH-Wert von 4,0 bis 5,0 durch Hinzugeben eines Säuerungsmittels durchgeführt wird, vorzugsweise wobei das Säuerungsmittel Citronensäure, Glucono-$\delta$-lacton, Adipat, Essigsäure und Kombinationen von beliebigen von ihnen umfasst.

4. Verfahren zur Bildung einer wässrigen Dispersion von Mikrokapseln nach Anspruch 1, wobei die Mikrokapseln 0,01-45 Gew.-% hydrophobe Substanz, 0,01-5 Gew.-% Proteinaggregate und 0,001-1 Gew.-% negativ geladenes Polymer umfassen.

5. Verfahren nach Anspruch 1, wobei die Mikrokapseln, wie in Anspruch 1 definiert, umfassen:

a. mindestens eine hydrophobe Substanz, umfassend Omega-3-Fettsäuren;
b. eine Grenzflächenschicht um die mindestens eine hydrophobe Substanz herum, wobei die Grenzflächenschicht umfasst:

i. Proteinaggregate, umfassend denaturiertes Molkenprotein, das zu mindestens 50 Gew.-% denaturiert ist, und
ii. Pektin.

6. Verfahren nach Anspruch 1, wobei die wässrige Dispersion, wie in Anspruch 1 definiert, die folgenden Merkmale aufweist:

- wobei die Proteinaggregate denaturiertes globuläres Protein, ausgewählt aus denaturierten Molkenproteinen, denaturierten Eiproteinen, denaturierten Sojaproteinen, denaturierten Lupinenproteinen, denaturierten Reisproteinen, denaturierten Erbsenproteinen, denaturierten Weizenproteinen, und Kombinationen von beliebigen von ihnen, umfassen,
- wobei die mindestens eine hydrophobe Substanz ausgewählt ist aus Lipiden, wasserunlöslichen Vitaminen, wasserunlöslichen Sterolen, wasserunlöslichen Flavonoiden, Aromen, etherischen Ölen, und Kombinationen von beliebigen von ihnen,
- wobei die mindestens eine hydrophobe Substanz eine Fettsäure, ausgewählt aus einer Omega-3-Fettsäure, einer Omega-6-Fettsäure, und Kombinationen von beliebigen von ihnen, umfasst,
- wobei die mindestens eine hydrophobe Substanz Docosahexaensäure (DHA), Eicosapentaensäure (EPA), Stearidonsäure, $\alpha$-Linolensäure (ALA), konjugierte Linolsäure (CLA), oder eine Kombination von beliebigen von ihnen umfasst,
- wobei das negativ geladene Polymer Pektin, Carboxymethylcellulose, modifizierte Stärken, Carrageenan, Alginat, teilweise deacetyliertes Xanthan, teilweise deacetyliertes Gellan, oder eine Kombination von beliebigen von ihnen umfasst.

7. Verfahren nach Anspruch 6, wobei die wässrige Dispersion, wie in Anspruch 1 definiert, die folgenden Merkmale aufweist:

- wobei das negativ geladene Polymer ausgewählt ist aus einem Pektin mit hohem Estergehalt, einem Pektin mit niedrigem Estergehalt, einem amidierten Pektin und Kombinationen von beliebigen von ihnen, oder

wobei das negativ geladene Polymer ein Pektin mit hohem Methylestergehalt umfasst, oder
wobei das negativ geladene Polymer ein Citruspektin umfasst.

8. Verfahren nach Anspruch 6, wobei die wässrige Dispersion, wie in Anspruch 1 definiert, die folgenden Merkmale aufweist:

- wobei die Proteinaggregate denaturiertes Molkenprotein umfassen,
- wobei die Proteinaggregate einen mittleren Durchmesser von 5-250 nm aufweisen,
- wobei die Mikrokapseln einen volumengewichteten mittleren Durchmesser im Bereich von 0,1-100 $\mu$m aufweisen,
- wobei die mindestens eine hydrophobe Substanz von zumindest einer Mehrheit der Mikrokapseln ein Öltröpfchen mit einem Durchmesser im Bereich von 0,01-20 $\mu$m ist,
- wobei die Grenzflächenschicht Proteinaggregate und negativ geladenes Polymer in einem Gewichtsverhältnis im Bereich von 10:1 bis 1:4 umfasst,
- wobei die Grenzflächenschicht Proteinaggregate und negativ geladenes Polymer in einer Menge von mindestens 55 % der Trockensubstanz umfasst,
- wobei die Grenzflächenschicht eine Dicke von 0,005-10 $\mu$m aufweist,
- wobei die mindestens eine hydrophobe Substanz mindestens 5 Gew.-% von der Mikrokapsel umfasst,
- wobei die mindestens eine hydrophobe Substanz Omega-3-Fettsäure umfasst, die Proteinaggregate Molkenproteinaggregate sind, und das negativ geladene Polymer ein Pektin mit hohem Methylestergehalt umfasst.

**Revendications**

1. Procédé de formation d'une dispersion aqueuse de microcapsules comprises dans un produit alimentaire, dans lequel le produit alimentaire a un pH de 1,0 à 5,5 et est une boisson de soda carbonatée, le procédé comprenant les étapes consistant à :

a) préparer une solution de protéine comprenant une protéine et de l'eau ;
b) chauffer la solution de protéine pour former une solution d'agrégats de protéine, les agrégats de protéine comprenant une protéine globulaire dénaturée qui est au moins à 50 % en poids dénaturée,
c) combiner la solution d'agrégats de protéine et une substance hydrophobe, et homogénéiser pour former une émulsion ;
d) ajuster le pH de l'émulsion à un pH de 4,0 à 5,0 ;
e) ajouter un polymère chargé négativement ayant une distribution de charge en bloc à l'émulsion et homogénéiser,
f) produire des microcapsules, comprenant de permettre au polymère chargé négativement et aux agrégats de protéine de s'accumuler à l'interface de la substance hydrophobe et de l'eau,

les microcapsules comprenant :
au moins une substance hydrophobe ; et

a. une couche d'interface autour de l'au moins une substance hydrophobe la couche d'interface comprenant :

i. des agrégats de protéine obtenus par traitement à la chaleur d'une solution de protéine aqueuse ; et
ii. un polymère chargé négativement ayant une distribution de charge en bloc,

les agrégats de protéine comprenant une protéine globulaire dénaturée qui est au moins à 50 % en poids dénaturée.

2. Procédé de formation d'une dispersion aqueuse de microcapsules selon la revendication 1, avec une ou plusieurs des caractéristiques suivantes :

- la solution de protéines comprenant des protéines globulaires ayant un poids moléculaire d'au moins 5 kDa,
- la solution de protéines comprenant 5 à 10 % en poids de protéines,
- le chauffage de la solution de protéines se produisant à une température de 60 °C à 200 °C,
- l'homogénéisation pour former une émulsion étant un processus d'homogénéisation en deux étapes à 800 et 80 bars.

3. Procédé de formation d'une dispersion aqueuse de microcapsules selon la revendication 1, dans lequel l'ajustement du pH de l'émulsion à un pH de 4,0 à 5,0 est effectué par addition d'un acidulant, de préférence l'acidulant comprenant l'acide citrique, la glucono delta-lactone, l'adipate, l'acide acétique et des combinaisons de n'importe lesquels d'entre eux.

4. Procédé de formation d'une dispersion aqueuse de microcapsules selon la revendication 1, dans lequel les micro-capsules comprennent de 0,01 à 45 % en poids de substance hydrophobe, 0,01 à 5 % en poids d'agrégats de protéine, et 0,001 à 1 % en poids de polymère chargé négativement.

5. Procédé selon la revendication 1, dans lequel les microcapsules telles que définies dans la revendication 1 comprennent :

    a. au moins une substance hydrophobe comprenant des acides gras oméga-3 ;
    b. une couche d'interface autour de l'au moins une substance hydrophobe la couche d'interface comprenant :

        i. des agrégats de protéine comprenant une protéine de blé dénaturée qui est au moins à 50 % en poids dénaturée, et
        ii. de la pectine.

6. Procédé selon la revendication 1, dans lequel la dispersion aqueuse telle que définie dans la revendication 1 a les caractéristiques suivantes :

    - les agrégats de protéines comprenant une protéine globulaire dénaturée choisie parmi les protéines de blé dénaturées, les protéines d'œuf dénaturées, les protéines de soja dénaturées, les protéines de lupin dénaturées, les protéines de riz dénaturées, les protéines de pois dénaturées, les protéines de blé dénaturées, et des combinaisons de n'importe lesquelles d'entre elles,
    - l'au moins une substance hydrophobe étant choisie parmi les lipides, les vitamines insolubles dans l'eau, les stérols insolubles dans l'eau, les flavonoïdes insolubles dans l'eau, les flaveurs, les huiles essentielles, et des combinaisons de n'importe lesquels d'entre eux,
    - l'au moins une substance hydrophobe comprenant un acide gras choisi parmi un acide gras oméga-3, un acide gras oméga-6 et des combinaisons de n'importe lesquels d'entre eux,
    - l'au moins une substance hydrophobe comprenant de l'acide docosahexaénoïque (DHA), de l'acide éicosapentaénoïque (EPA), de l'acide stéaridonique, de l'acide $\alpha$-linolénique (ALA), de l'acide linoléique conjugué (CLA) ou une combinaison de n'importe lesquels d'entre eux,
    - le polymère chargé négativement comprenant de la pectine, de la carboxy méthyl cellulose, des amidons modifiés, du carraghénane, de l'alginate, du xanthane partiellement désacétylé, du gellane partiellement désacétylé, ou une combinaison de n'importe lesquels d'entre eux.

7. Procédé selon la revendication 6, dans lequel la dispersion aqueuse telle que définie dans la revendication 1 a les caractéristiques suivantes :

    - le polymère chargé négativement étant choisi parmi une pectine à ester élevé, une pectine à ester faible, une pectine amidée et des combinaisons de n'importe lesquelles d'entre elles, ou

le polymère chargé négativement comprenant une pectine à ester de méthyle élevé ou le polymère chargé négativement comprenant une pectine de citron.

8. Procédé selon la revendication 6, dans lequel la dispersion aqueuse telle que définie dans la revendication 1 a les caractéristiques suivantes :

    - les agrégats de protéine comprenant une protéine de blé dénaturée,
    - les agrégats de protéine ayant un diamètre moyen de 5 à 250 nm,

EP 2 811 846 B2

- les microcapsules ayant un diamètre moyen en poids en volume dans la plage de 0,1 à 100 $\mu$m,
- l'au moins une substance hydrophobe d'au moins une majorité des microcapsules étant une gouttelette d'huile ayant un diamètre dans la plage de 0,01 à 20 $\mu$m,
- la couche d'interface comprenant des agrégats de protéine et un polymère chargé négativement dans un rapport en poids dans la plage de 10 : 1 à 1 : 4,
- la couche d'interface comprenant des agrégats de protéine et un polymère chargé négativement en une quantité d'au moins 55 % de matière sèche,
- la couche d'interface ayant une épaisseur de 0,005 à 10 $\mu$m,
- l'au moins une substance hydrophobe comprenant au moins 5 % en poids de la microcapsule,
- l'au moins une substance hydrophobe comprenant un acide gras oméga-3, les agrégats de protéine étant des agrégats de protéine de blé et le polymère chargé négativement comprenant une pectine à ester de méthyle élevé.

**EP 2 811 846 B2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090004333 A1

- EP 2292102 A1